# EUROPEAN PATENT SPECIFICATION

(11) **EP 0 981 359 B1**
(45) Date of publication and mention of the grant of the patent: **19.03.2003**
(21) Application number: 98921528.0
(22) Date of filing: 18.05.1998
(51) Int. Cl.: A61K 38/04, A61K 38/13, A61K 38/21, A61P 37/06

(54) **COMPOSITIONS FOR ENHANCING IMMUNOSUPPRESSANTS' PHARMACOLOGICAL ACTIVITIES**
ZUSAMMENSETZUNGEN ZUR VERSTÄRKUNG DER PHARMAKOLOGISCHEN AKTIVITÄT VON IMMUNOSUPPRESSIVA
COMPOSITIONS STIMULANT L'ACTIVITE PHARMACOLOGIQUE DES IMMUNOSUPPRESSEURS

(30) Priority: 19.05.1997 FI 972121
(43) Date of publication of application: 01.03.2000
(73) Proprietor: Zavialov, Vladimir Petrovich, Moscow Region, Lyubuchany, 142380 (RU); Vasilenko, Raisa Nikolaevna, Moscow Region, Lyubuchany, 142380 (RU); Dolgikh, Dmitry Aleksandrovich, Moscow, 113093 (RU); Kirpichnikov, Mikhail Petrovich, Moscow, 113000 (RU); Navolotskaya, Elena Vitalievna, Pushchino, 142292 (RU); Korpela, Timo Kalevi, 20500 Turku (FI)
(72) Inventor: Zavialov, Vladimir Petrovich, Moscow Region, Lyubuchany, 142380 (RU); Vasilenko, Raisa Nikolaevna, Moscow Region, Lyubuchany, 142380 (RU); Dolgikh, Dmitry Aleksandrovich, Moscow, 113093 (RU); Kirpichnikov, Mikhail Petrovich, Moscow, 113000 (RU); Navolotskaya, Elena Vitalievna, Pushchino, 142292 (RU); Korpela, Timo Kalevi, 20500 Turku (FI)
(74) Representative: Karvinen, Leena Maria
(86) International application number: FI9800418
(87) International publication number: WO98052594

(56) References cited:
- WO-A-90/09806
- WO-A-94/10313
- FR-A- 2 706 772
- DIALOG INFORMATION SERVICES, File 155, Medline, Dialog Accession No. 08309301, Medline Accession No. 95272551, ZAV'YALOV V.P. et al., "The Sequence 130-137 of Human Interferon-alpha 2 is Involved in the Competition of Interferon, Prothymosin alpha and Cholera Toxin B Subunit for Common Receptors on Human Fibroblasts"; & MOL. IMMUNOL., April 1995, 32 (6), p. 425-31.
- DIALOG INFORMATION SERVICES, File 73, Embase, Dialog Accession No. 10073054, ZAROGOULIDIS K. et al., "Interferon alpha-2a and Combined Chemotherapy as First Line Treatment in SCLC Patients: A Randomised Trial"; & LUNG CANCER, 1996, 15/2 (197-205).

## Description

### FIELD OF INVENTION

This invention is related to human or animal medicine and more specifically to improving the therapeutic effects of common immunosuppressive, anti-inflammatory and/or antiparasitic drugs where the effective ingredients contain cyclosporins, FK506 or rapamycin. This invention provides new compositions for efficient amplification of the activities of the said components to decrease their therapeutic dose to avoid their side effects.

### BACKGROUND OF INVENTION

Cyclosporins are cyclic peptides composed of eleven amino acid residues which include previously unknown N-methylated amino acid residues. Cyclosporins are commercially important products isolated from the culture broths of the fungal species *Tolypocladium.* Cyclosporins are applied to prolong survival of allogenic transplants involving skin, kidney, pancreas, bone marrow, small intestine and lung. The efficacy of cyclosporins is presently known to be due to the specific and reversible inhibition of immunocompetent cells, primarily T-helper cells. Unfortunately, cyclosporins' systemic use have been associated with a high incidence of renal toxicity (kidney failure), some cases of hepatotoxicity, increased incidence of lymphoid tumors and increased incidence of opportunistic infections. The side effects of cyclosporins are so severe and so common that they limit their applications to life-threatening or severe sight-threatening diseases.

Cyclosporins, like FK506 and other related molecules, bind specifically to certain physiologically important molecular species in tissues and blood circulation (Denesyuk A. et al., 1993, Biochem. Biophys. Res. Commun. 197, pp. 1438-1442; Denesyuk A. et al., 1993, Biochem. Biophys. Res. Commun. 194, pp. 280-286; Denesyuk A. et al., 1993, Biochem. Biophys. Res. Commun. 192, pp. 912-917). After identification of cyclosporin A, other cyclosporins, B, C, D, E, and G, containing minor differences in the molecular structure of cyclosporins have been reported. Many of these cyclosporins exhibit pharmaceutical utility related to cyclosporin A and the literature involves abundantly examples and suggestions for their use as therapeutic agents.

Cyclosporins were first proposed for be used as an antifungal agents, but their immunosuppressive action was found to be of more importance. Cyclosporins specifically and reversibly inhibit immunocompetent cells, primarily T-helper cells, by specific interactions with peptidyl-prolyl *cis-trans* isomerase (PPIase; see, Schreiber S. and Crabtree G., 1992, Immunol. Today 13, pp. 136-142; Zav'yalov V. et al., 1995, Acta Pathol. Microbiol. Immunol. Scand. 103, pp. 401-415). These multifunctional PPIase proteins may also serve as ATP-independent molecular chaperones facilitating the folding of target proteins and assembly of multisubunit protein complexes (Schmid F. et al., 1993, Adv. Protein Chem. 46, pp. 25-65), but their primary function is the binding of the immunosuppressive drugs cyclosporin A, FK506 and rapamycin. The PPIases with such activity were termed immunophilins (ImP's). These protein receptors fall into two major groups for their structure: the cyclosporin binding proteins, also termed cyclophilins (CyPs), and the FK506 binding proteins, termed FKBPs, some of the latter preferentially binding rapamycin (Trandini C. et al., 1992, FASEB J. 6, pp. 3410-3420; Liu J., 1993, Immunol. Today 14, pp. 290-295). It has been found that Ca²⁺ and calmodulin-dependent protein phosphatase, calcineurin (CaN), binds with high affinity to biologically active ImP-drug complexes. The experimental data suggest that the cytoplasmic NF-AT protein (NF-ATc) is a substrate, either direct or indirect, via a phosphatase cascade for CaN. NF-AT is a complex protein made of at least two subunits. One subunit of NF-AT (NF-ATp), a DNA-binding phosphoprotein, is a substrate for CaN *in vitro* and interacts with Fos and Jun proteins in the nucleus of activated T cells (Jain J. et al., 1993, Nature 365, pp. 352-355). Dephosphorylation by CaN might directly induce nuclear translocation of NF-ATc. The active form of this protein interacts with functional sequences within the IL-2 enhancer. According to a hypothesis, the complexes cyclosporin A-CyP or FK506-FKBP-12 block nuclear translocation of NF-ATc and activation of IL-2 gene expression due to the inhibition of CaN activity.

Cyclosporins' immunosuppressive properties have led to their wide routine usage in immune system-related diseases, as described, for example, in Finnish patent publication 900604. Correspondingly, the number of patents for these purposes on various aspects of cyclosporins is large. In addition, eye medicine has benefited from cyclosporins. The U.S. Patent No. 4, 649, 047 describes a method for the treatment of phacoanaphylactic endophthalmitis and uveitis in the anterior or posterior segment of an eye wherein cyclosporin is topically administered to the eye. In other ophthalmic applications, cyclosporin has been used topically only for the treatment of external (e.g., corneal) eye diseases. BenEzra et al., 1986, Amer. J. Ophthlmol. 101, pp. 278-282 described the effect of 2% cyclosporin eyedrops on severe vernal keratoconjuctivitis which is a seasonal allergic disorder unrelated to tear deficiency. Hunter et al., 1981, Clin. Exp. Immunol. 45, pp. 173-177 described the topical administration of cyclosporin in a rabbit model of corneal graft rejection with positive results. Boisjoly et al., 1984, Arch. Ophthalmol. 102, pp. 1804-1807 reported that topical application of cyclosporin had a beneficial prophylactic effect towards the treatment of severe herpetic stromal keratitis. Mosteller et al., 1984, Investigative Ophthalmol. Supp. 25, pp. 3-38 treated corneal allograft rejection in rabbits by applying a single dose of a 10% cyclosporin A oinment in the lower culde-sac of the eyelids. Cyclosporins have also been used systemically in other ophthalmic applications, where the disease being treated is not limited to the eye surface. For example, Nussenblatt et al., 1983, Amer. J. Ophthalmol. 96, pp. 275-282 reported clinical improvement in some patients with noninfectious posterior uveitis following systemic treatment with cyclosporin.

Cyclosporins are primarily administered orally or by injection. Unfortunately, cyclosporins used systemically have been associated with a high incidence of renal toxicity (kidney failure), some cases of hepatotoxicity, increased incidence of lymphoid tumors and increased incidence of opportunistic infections. Cyclosporin are only slightly less toxic than other immunosuppressive agents such as cytoxan or aziothioprine. The systemic side effects of cyclosporins are so severe and so common that they limit cyclosporins use to life-threatening or in some cases severe sight-threatening diseases.

As described in U.S. Patent No. 4, 117, 118 1978 ( Harri et al.), cyclosporin is readily soluble in most of the usual organic solvents and practically insoluble in petroleum ether and water. Poor solubility of cyclosporins makes absorption in intestine with oral administration as well as actions in intravenous applications variable from patient to patient. If rather high and constant level of cyclosporins are required in the blood circulation, administration of cyclosporins is complex. As distributed by Sandoz Ltd. under the tradename Sandimmune, cyclosporin for oral administration is dissolved in olive oil for further dilution with food and in polyoxyethylated castor oil and ethanol for intravenous injection. The release of cyclosporins from the carrier substances is patient-dependent where either the dose is not therapeutically effective or it is toxic. As stated, prolonged systemic administration of cyclosporins associated with transplantation and related uses creates risks for other diseases including cancers. To be able to use considerably lower than toxic level of effective cyclosporin doses is therefore of key importance.

Recently, the attention was brought to the long-neglected role of type I interferons (IFN-α,β,ω,τ) in the modulation of immune response (Belardelli F. and Gresser I., 1996, Immunol. Today 17, pp. 369-372). The immunomodulating activity of IFN-a,b,w,t is very complex and includes:
- inhibition of lymphocyte proliferation after stimulation with lectins or allogenic cells;
- prolongation of skin grafts;
- inhibition of delayed type of hypersensitivity;
- differentiation towards a Thl type of immune response;
- stimulation *in vivo* of long-lived antigen-specific memory CD8+ cell cytotoxicity;
- inhibition of suppressor T cells;
- enhancement of natural killer (NK) cell cytotoxicity;
- enhancement of major histocompatibility complex (MHC) class I antigens expression.

First three immunomodulating properties of type I IFNs (inhibition of lymphocyte proliferation after stimulation with lectins or allogenic cells, prolongation of skin grafts, and inhibition of delayed type of hypersensitivity) are useful for amplification of immunosuppressants' (cyclosporins, FK506 and rapamycin) activity. But other immunomodulating properties of type I IFNs (differentiation towards a Thl type of immune response; stimulation *in vivo* of long-lived andgen-specific memory CD8⁺ cell cytotoxicity; inhibition of suppressor T cells; enhancement of NK cell cytotoxicity; enhancement of MHC class I antigens expression) can induce an opposite effect Indeed, it was observed that a monoclonal antibody directed against the extracellular domain of the human (h)IFN-α receptor, which inhibits both the binding and biological activity of all the type I IFNs tested, exerted a dose-dependent inhibition of the mixed lymphocyte reaction and induced permanent survival of skin allografts in MHC-divergent Cynomologus monkeys treated with a subeffective dose of cyclosporin A ( Tovcy, M.G. et al, 1996, J. Leukocyte Biol. 59, pp. 512-517).

In the present invention we suprisingly observed that the physiological effects of immunosuppressants (cyclosporins, FK506 and rapamycin) can be dramatically increased by the simultaneous administration of any one of the immunosuppressants and the peptide corresponding to the high-affinity binding/anti-lymphoproliferative site of IFN-α or the recombinant protein having the amino acid sequence corresponding to the said site. Peptides are specially favourable additives since they do not cause harmful symptoms and the acitivity quenching due to the generation of antibodies against larger polypeptides such as natural interferons.

Long time ago there was discovered the primary structure homology of hIFNs-α/β and thymus hormone - thymosin α₁ (TMα₁) (Zav'yalov V. and Denesyuk A., 1984, Doklady Akademii Nauk SSSR 275, pp. 242-246). Comparison of the primary structures of proTMα and hIFNs-α/β reveals a homology of the prohormone with the IFNs' sites making up the C-terminal helices D and E, however, the highest homology is observed for the C-terminal part of helix D and the N-terminal part of loop DE (Zav'yalov V. et al., 1989, Immun. Lett. 22, pp. 173-182; Zav'yalov V. et al., 1990, Biochim. Biophys. Acta 1041, pp.178-185). One of the synthesized peptides (α-peptoferon) overlapping the TMα₁-like sequence of hIFN-α2, effectively competed with hIFN-α2, TMα₁, proTMα for common receptors on mouse thymocytes (i.e. the Kᵢ of recombinant (r)hIFN-α2 binding by α-peptoferon is equal to about 10⁻¹² M) (Zav'yalov V. et al., 1991, FEBS Lett. 278, pp. 187-189; Zav'yalov V. et al., 1995, Molec. Immun. 32, pp. 425-431). Recently the first example of successful grafting of hIFN-α2's TMα₁-like site to the design *de novo* protein albeferon was described (Dolgikh D. et al., 1996, Protein Engin. 9, pp. 195-201). The IFN-α2 fragment corresponded to the TMα₁-like sequence 130-137 was inserted into the N-termini of an albebetin molecule just after the initiatory Met residue. The chimeric protein (albeferon) was expressed in a wheat germ cell-free translation system and tested for its compactness and stability. It has been shown that albeferon is practically as compact as natural proteins of corresponding molecular weight and possesses high stability toward the urea-induced unfolding. To testify the affinity of albeferon to murine thymocyte receptor, the protein inhibitory effect on the binding of radiolabeled [¹²⁵I] a-peptoferon to the receptors has been studied. The albeferon competitive inhibition coefficient (IC₅₀) and the calculated inhibition constant (Kᵢ) are very close to that of rhIFN-α2. It was demonstrated that cell surface binding characteristics correlate with consensus type I IFN enhanced anti-lymphoproliferative activity on the human periferal polymorphonuclear cells (Klein S. et al., 1996, J. Interferon and Cytokine Res. 16, pp. 1-6: Dhib-Jalbut S. et al., 1996, J. Interferon and Cytokine Res. 16, pp. 195-200).

The present invention includes the compositions for efficient amplification of immunosuppressive activity of cyclosporins, FK506 or rapamycin by peptides corresponding to the high-affinity binding/anti-lymphoproliferativc site of IFNs-α,β,ω,τ or recombinant proteins having the amino acid sequences corresponding to the said site to decrease therapeutic dose of the both groups of compounds, and as the consequence to avoid their undesirable side effects during organ and tissue transplantation, and treatment of different diseases.

### SUMMARY OF THE INVENTION

The present invention provides the compositions for efficient amplification of immunosuppressive activity of cyclosporins, FK506 or rapamycin to decrease therapeutical dose of immunosuppressants, and as the consequence to avoid their undesirable side effects during organ and tissue transplantation, and treatment of different diseases.

These compositions include cyclosporins, FK506 or rapamycin and biologically active peptides corresponding to the high-affinity binding/anti-lymphoproliferative site of IFNs-α,β,ω,τ, or recombinant proteins having the amino acid sequences corresponding to the said site.

Specifically the invention concerns compositions consisting of cyclosporins, FK506 or rapamycin with one or more of the peptides containing at least 5 identical amino acid residues in the following table:

| | | | | | | | |
|---|---|---|---|---|---|---|---|
| L | T | E | K | K | Y | S | P |
| | R | R | R | R | H | R | R |
| | Q | D | N | D | | D | D |
| | M | L | M | N | | N | L |
| | | I | S | S | | S | |
| | E | A | E | | | A | |
| | G | | | | | | |

wherein,
each amino acid residue in the top sequence can be modified with any of the amino acid residues under it.

### BRIER DESCRIPTION OF THE DRAWINGS

FIG. 1 Effect of rhIFN-α2 and of the peptide corresponding to the high-affinity binding/anti-lymphoproliferative site of hIFN-α2 (α-peptoferon) on mitogen (PHA)-driven proliferation of human peripheral polymorphonuclear cells.

FIG. 2 Effect of rhIFN-α2 and of the peptide corresponding to the high-affinity binding/anti-lymphoproliferative site of hIFN-α2 (α-peptoferon) on the proliferation of an IFN-sensitive human T-lymphoblastoid cell line MT-4.

FIG. 3 Effect of rhIFN-α2, albeferon and albebetin on mitogen (PHA)-driven proliferation of human peripheral polymorphonuclear cells.

FIG. 4 Effect of rhIFN-α2, albeferon and albebetin on the proliferation of an IFN-sensitive human T-lymphoblastoid cell line MT-4.

FIG. 5 Effect of rhIFN-α2 and of the peptide corresponding to the high-affinity binding/anti-lymphoproliferative site of hIFN-α2 (α-peptoferon) on mitogen (PHA)-driven proliferation of human peripheral polymorphonuclear cells in the presence of 20 mM cyclosporin A in cultural medium.

FIG. 6 Effect of albeferon on mitogen (PHA)-driven proliferation of human peripheral polymorphonuclear cells in the presence of 10 mM cyclosporin A in cultural medium.

### DETAILED DESCRIPTION OF THE INVENTION

Cyclosporins, FK506 and rapamycin are widely used commercially important drugs for treatment of various diseases of human and higher animals. However, the use of these drugs have severe drawbacks for their toxicity and narrow effective dose range. This forms the basis of either developing other more effective and less toxic related drugs or increasing their activity by other ingredients. The latter has been attempted only by making drug formulations which provide either more effective absorption in intestine or prolonged delivery in intestine or in intravenous administration.

In this invention, there was surprisingly observed that certain favourable physiological effects of cyclosporins, FK506 and rapamycin on human cells can be amplified by biologically active peptides corresponding to the high-affinity binding/anti-lymphoproliferative site of IFNs-α,β,ω,τ or recombinant proteins having the amino acid sequences corresponding to the said site. The peptides and recombinant proteins mentioned above can be readily produced by widely available synthetic or recombinant techniques. The present invention provides definite improvement of compositions for drugs based on cyclosporins, FK506 or rapamycin aimed for human and higher animals. These compositions include the immunomodulators and biologically active peprides corresponding to the high-affinity binding/anti-proliferative site of IFNs-α,β,ω,τ or recombinant proteins having the amino acid sequences corresponding to the said site. Although all the potentially bioactive peptides or the recombinant proteins containing the amino acid sequences of the peptides were not possible to test experimentally here with the immunomodulator drugs (cyclosporins, FK506 and rapamycin) such structures were revealed by extensive comparisons of available amino acid sequences of interferons from different species by computer and molecular modelling techniques taking into account the experimental data on the localisation of the high-affinity binding/anti-lymphoproliferative site of IFNs-α,β,ω,τ and the experimental data on the competition of type I IFNs for the common receptors. The peptides according to this invention have the advantage of not give rise to antibodies like large polypeptides and natural interferons and thus pepetides *as* such or bound to carrier molecules can be used for long periods.

In the previous studies (Zav'yalov V. et al., 1991, FEBS Lett. 278, pp. 187-189; Zav'yalov V. et al., 1995, Molec. Immun. 32, pp. 425-431) it was demonstrated that only octapeptide corresponding to the 130-137 amino acid residues of hIFN-α2 had the same or higher affinity to the specific receptors in comparison with recombinant hIFN-α2. It is well-known that all type I IFNs compete for the common receptors and can induce the common type I IFN activities. Therefore, it is reasonable to assume that in the process of natural selection the changes in the amino acid sequence of the high-affinity binding/anti-lymphoproliferative site of IFNs-α,β,ω,τ were selected not to abolish the biological activities of the site. Consequently, all type I natural and recombinant IFNs as well as peptides corresponding to their high-affinity binding/anti-lymphoproliferative site, and recombinant proteins having the amino acid sequences corresponding to the said site might reproduce the anti-proliferative activity and amplify immunosuppressive activity of cyclosporins.
For the testing of synergism of cyclosporin, FK506 or rapamycin and peptides corresponding to the high-affinity binding/anti-lymphoproliferative site of INFs-α,β,ω,τ or recombinant proteins having the amino acid sequences corresponding to the said site we employed the classic anti-lymphoproliferative test system with human peripheral polymorphonuclear cells and human T-lymphoblastoid cell line MT-4. The cell culture conditions and the conditions of the humoral cells in blood circulation are closely related. In fact, in cell cultures, which are commonly used for testing of potential drugs, the conditions are strictly maintained similar to the blood circulation as to the temperature, pH, buffer, minerals, CO₂ and O₂ partial pressures and so on. On the other hand, in this special case the target cells of cyclosporins or the other immunosuppressants and the peptides corresponding to the high-affinity binding/anti-lymphoproliferative site of IFNs-α,β,ω,τ or recombinant proteins having the amino acid sequences corresponding to the said site being used as the drugs exist specifically in the blood circulation in very equal conditions to the cell cultures. Thus, it is highly predictable that the drug compositions of the present invention can be used as medical drugs for purposes previously used for immunosuppressants alone as the active ingredient in the drug formulations.

While the main experimental proof of the present invention lies on the use of peptides corresponding to the high-affinity binding/anti-lymphoproliferative site of IFNs-α,β,ω,τ or recombinant proteins having the amino acid sequences corresponding to the said site with cyclosporins, also FK506 and rapamycin can be applied with related amplification effect as with cyclosporins. Although the present invention describes effects of peptides corresponding to the high-affinity binding/anti-lymphoproliferative site of IFNs-α,β,ω,τ or recombinant proteins having the amino acid sequences corresponding to the said site on immunosuppressants such as cyclosporins, FK506 and rapamycin, it is evident that the peptides corresponding to the high-affinity binding/anti-lymphoproliferative site of IFNs-α,β,ω,τ or recombinant proteins having the amino acid sequences corresponding to the said site will increase equally well the activities of any other immunosuppressant.

### Example 1.

Ninety five ml of blood were taken from each of 10 volunteer donors, and 5 ml of 3-5% EDTA were added to the blood immediately and mixed carefully to prevent coagulation. The blood was than mixed with the equal volume of medium 199 or with buffered physiological salt solution. Ficoll-Paque (Pharmacia) was poured into plastic centrifuge test-tubes with caps (Costar) (3 ml of Ficoll-Paque per each test-tube, volume of a test-tube - 15 ml, diameter - 13 mm at room temperature, and then 6-7 ml of diluted blood were carefully layered on top). The test-tubes were centrifuged at 20 °C for 40 minutes, at 400 g, with the brake of centrifuge being switched off. The mononuclear cells formed a layer on the border of the liquid division and the cells were collected with a Pasteur pipette into a centrifuge test-tube with 5 ml of RPMI 1640, containing 2% of inactivated fetal serum and penicillin (100 units/ml) with streptomycin (100 units/ml). Then the cells were washed with physiological buffer solution (PBS) three times with centrifuging at 400 g for 5 minutes at the temperature of +8 °C. When the concentration of the cells was 2.5^{·}10⁶ cells/ml, they were placed in RPMI 1640 medium, containing 5% of fetal serum (heated for 30 minutes at 56 °C), 100 units/ml of penicillin, 100 units/ml of streptomycin, 1 ml 200 mM of L-glutamine per 100 ml of serum. All the reagents were produced by Gibco. The cells were dropped into a 96-well plate (100 µl per each plate) with mitogen - phytohemagglutinin (PHA) (final concentration - 2 mg/ml), the preparations under study and immunosuppressants (cyclosporin A, FK506 or rapamycin) in concentration - 5-20 mM) were pipetted into the tubes beforehand. The plate was placed in a CO₂-incubator at 5% CO₂ with humid atmosphere for 72 hours. 12-18 hours before the completion of the incubation 1 mCi of [³H]-thymidine was added into each well. Then the cells were washed on a semiautomatic harvester (Titertec Flow Lab.) using filters (Whatman). Each filter was carefully transferred into bottles, containing 5 ml of scintillation liquid (Sigma). The number of disintegrations per minute was counted using a Minibeta counter (LKB). The stimulation index was determined by arithmetical mean of at least three parallel measurements ±SD to the corresponding control.

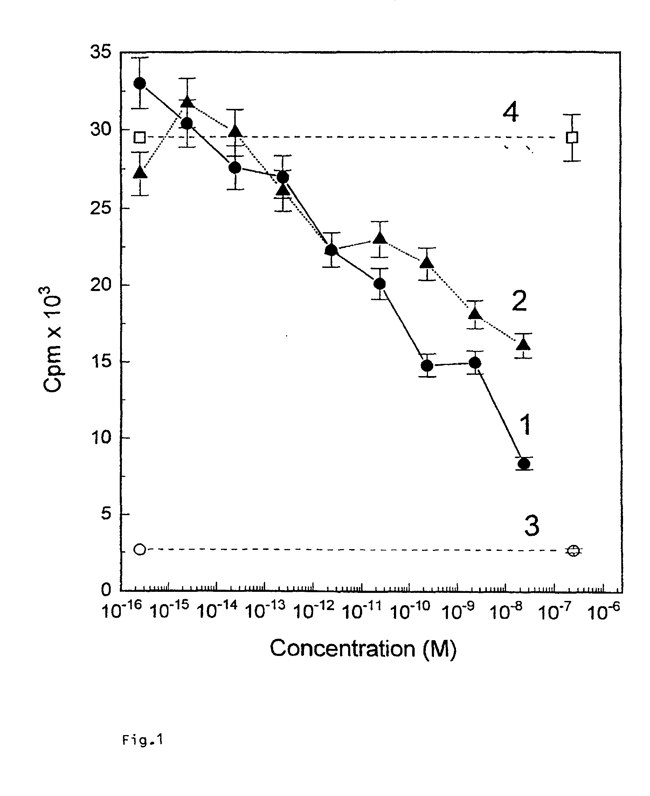

Fig. 1 displays, respectively, the effect of rhIFN-α2 and of the peptide corresponding to the high-affinity binding/anti-lymphoproliferative site of hIFN-α2 (α-peptoferon) on mitogen (PHA)-driven proliferation of human peripheral polymorphonuclear cells. PHA induced strong lymphoproliferative response that was inhibited by rhIFN-α2 and a-peptoferon in a dose-dependent fashion. rhIFN-α2 and α-pentoferon demonstrated comparable effects. rhIFN-γ had either no effect or a minimal enhancing effect on the lymphoproliferative response. (1) rhIFN-α2; (2) α-peptoferon; (3) control: cultural medium; (4) control: PHA.

### Example 2.

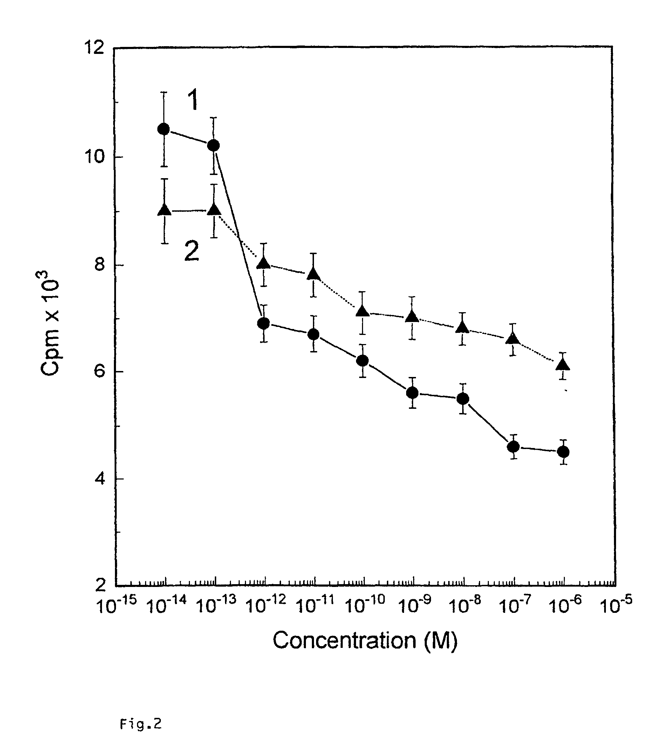

Fig. 2 shows the effect of rhIFN-α2 and of the peptide corresponding to the high-affinity binding/anti-lymphoproliferative site of hIFN-α2 (α-peptoferon) on the proliferation of an IFN-sensitive human T-lymphoblastoid cell line MT-4. The proliferation of human T-lymphoblastoid cell tine MT-4 was inhibited by rhIFN-α2 and a-peptoferon in a dose-dependent fashion. rhIFN-α2 and a-peptoferon demonstrated comparable effects. Similar methods as in Example 1 were employed. (1) rhIFN-α2; (2) α-peptoferon.

### Example 3.

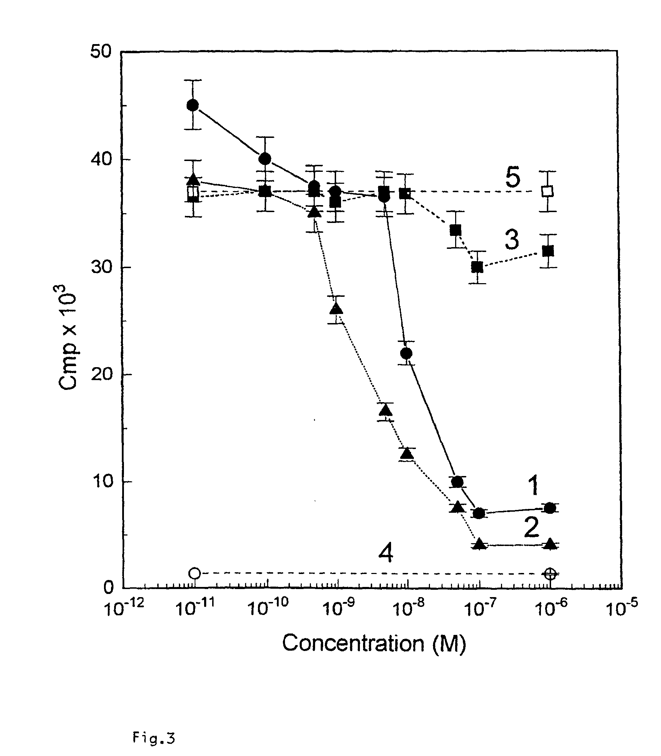

Fig. 3 displays the effect of rhIFN-α2 and the designed *de novo* chimesic protein albeferon containing the TMα₁-like sequence 130-137 of the hIFN-α2 inserted into the N-termini of the protein just after the initiatory Met residue (2), and the designed *de novo* protein albebetin without the hIFN-α2 fragment (3) on PHA-driven proliferation of human peripheral polymorphonuclear cells. PHA induced strong lymphoproliferative response that was inhibited by rhIFN-α2 and albeferon in a dose-dependent fashion. rhIFN-α2 and albeferon demonstrated comparable effects. Albebetin had no effect on the lymphoproliferarive response. The results show that the hIFN-α2 fragment corresponded to the TMα₁-like sequence 130-137 is responsible for the anti-lymphoproliferative effect of rhIFN-α2. Similar methods as in Example 1 were employed. (1) rhIFN-α2; (2) albeferon; (3) albeberin; (4) control: cultural medium; (5) control: PHA

### Example 4.

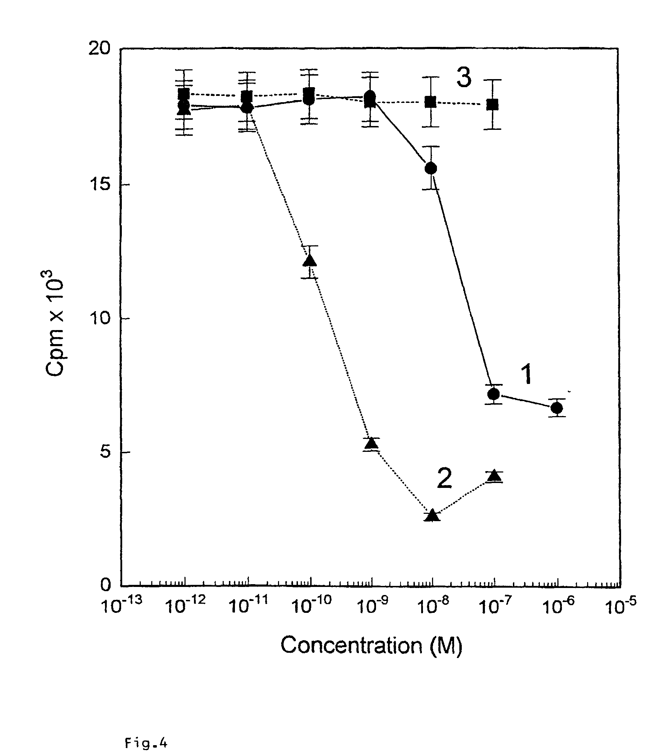

Fig. 4 demonstrates the effect of rhIFN-α2, albeferon and albebetin on the proliferation of an IFN-sensitive human T-lymphoblastoid cell line MT-4. The proliferation of human T-lymphoblastoid cell line MT-4 was inhibited by rhIFN-α2 and albeferon in a dose-dependent fashion. In this experiment albeferon had higher activity than the sample of rhIFN-α2 used but comparable with other data on rhIFN-α2 (see Figs. 1-2 and the data described in: Dhib-Jalbut. S. et al., 1996, J. Interferon and Cytokine Res. 16, pp. 195-200). Albebetin had no effect on the lymphoproliferative response. The results show that the hTFN-α2 fragment corresponded to the TMα₁-like sequence 130-137 is responsible for the anti-lymphoproliferative effect of rhIFN-α2. (1) rhIFN-α2; (2) albeferon; (3) albebetin.

### Example 5.

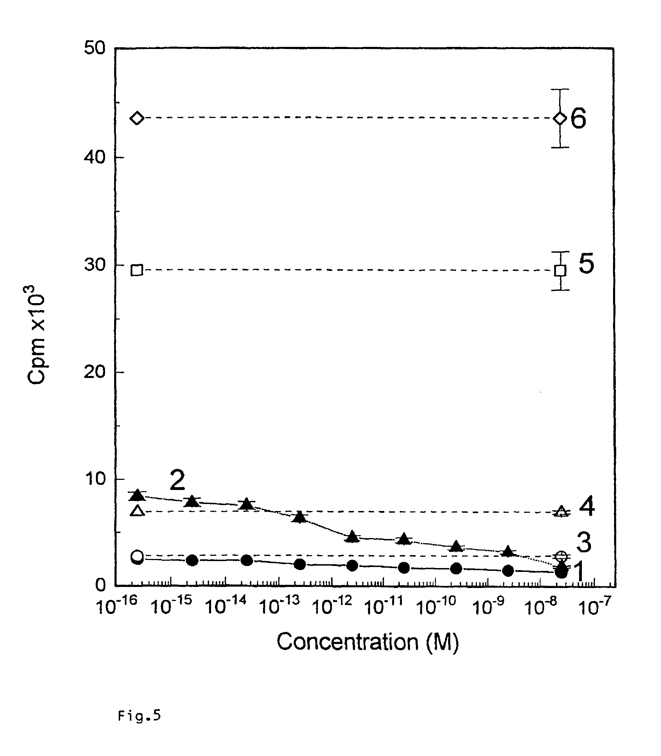

Fig. 5 displays the effect of rhIFN-α2 on PHA-driven proliferation of human peripheral polymorphonuclear cells in the presence of 20 mM cyclosporin A (CsA) in cultural medium. PHA induced strong lymphoproliferative response. The selected concentration of CsA had enhancing effect on the lymphoproliferative response. The administration of extremely low amount of rhIFN-α2 (10⁻¹⁶ M) totally abolished lymphoproliferative response induced by PHA. The comparable effect on mitogen-driven proliferation of peripheral blood human T-lymphocytes in the presence of 20 mM CsA in cultural medium was observed for the octapeptide corresponding to the high-affinity binding/anti-lymphoproliferative site of hIFN-α2 (α-peptoferon). The results demonstrate strong synergism of anti-lymphoproliferative action of CsA and rhIFN-α2 or the peptide corresponding to the high-affinity binding/anti-lymphoproliferative site of hIFN-α2 (α-peptoferon). Related results were obtained with 1-50 mM concentration of FK506 or rapamycin instead of cyclosporin A. Similar methods as in Example 1 were employed. (1) rhIFN-α2 and 20 mM CsA; (2) α-peptoferon and 20 mM CsA; (3) control: cultural medium; (4) control: 20 mM CsA; (5) control: PHA; (6) control: 20 mM CsA and PHA.

### Example 6.

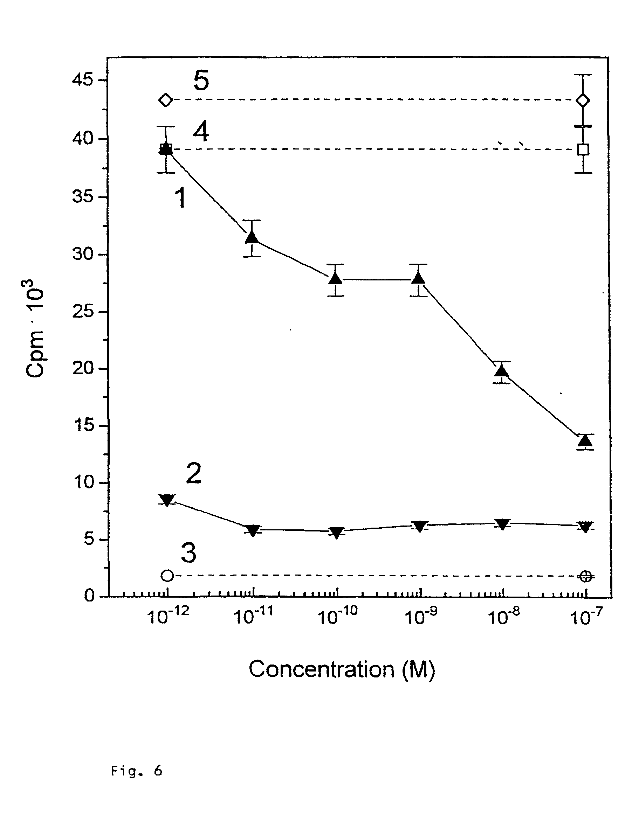

Fig. 6 displays the effect of albeferon on PHA-driven proliferation of human peripheral polymorphonuclear cells in the presence of 10 mM cyclosporin A (CsA) in cultural medium. PHA induced strong lymphoproliferative response. The selected concentration of CsA had enhancing effect on the lymphoproliferative response. The administration of extremely low amount of albeferon (10⁻¹² M) totally abolished lymphoproliferative response induced by PHA. Related results were obtained with 1-50 mM concentration of FK506 or rapamycin instead of cyclosporin A. Fig. 6 also shows that the octapeptide corresponding to the high-affinity binding/anti-lymphoproliferative site of hIFN-α2 (α-peptoferon) which was genetically immobilized on the macromolecular carrier (i.e. on the *de novo* protein albebetin) is biologically active. Similar methods as in Example 1 were employed. (1) albeferon; (2) albeferon and 10 mM CsA; (3) control: cultural medium; (4) control: PHA; (5) control: PHA and 10 mM CsA.

## Claims

1. A drug composition consisting of immunosuppressants cyclosporins, FK506, or rapamycin and the bioactive peptides corresponding to the high-affinity binding/anti-lymphoproliferative site of interferons α,β,ω,τ, or recombinant proteins carrying one or more of the sequences corresponding to the structures of the bioactive peptides corresponding to the high-affinity binding/anti-lymphoproliferative site of the said interferons for the aim of amplification of immunosuppressants' activities to decrease their therapeutic dose, and as the consequence to avoid their undesirable side effects during organ and tissue transplantation or during treatment of different diseases, wherein cyclosporins, FK506 or rapamycin can be exploited.

2. The composition according to Claim 1 consisting of immunosuppressants cyclosporins, FK506 or rapamycin with one or more of the peptides containing at least 5 identical amino acid residues in the following table:
| | | | | | | | |
|---|---|---|---|---|---|---|---|
| L | T E | | K | K | Y | S | P |
| | R | R | R | R | H | R | R |
| | Q | D | N | D | | D | D |
| | M | L | M | N | | N | L |
| | | I | S | S | | S | |
| | E | A | E | | | A | |
| | G | | | | | | |
wherein,
each amino acid residue in the top sequence can be modified with any of the amino acid residues under it.

3. The composition according to Claim 1 consisting of immunosuppressants cyclosporins, FK506 or rapamycin, and recombinant proteins carrying one or more of the sequences corresponding to the peptide variation of Claim 2.

4. The composition according to Claim 2 consisting of immunosuppressants cyclosporins, FK506 and rapamycin, and the bioactive peptides wherein the active peptides are genetically or chemically modified or genetically or chemically or physically bound to a small-molecular or macromolecular substance for the aim of increasing the stabilities of the peptides in physiological conditions or for regulating the bioavailability of the said peptides.

## Patentansprüche

1. Arzneistoffzusammensetzung, bestehend aus den Immunsuppressiva Cyclosporine, FK506 oder Rapamycin und den bioaktiven Peptiden, die der mit hoher Affinität-Bindungs-/anti-lymphoproliferativen Stelle der Interferone α, β, ω, τ entsprechen, oder rekombinanten Proteinen, die eine oder mehrere der Sequenzen tragen, die den Strukturen der bioaktiven Peptide entsprechen, die der mit hoher Affinität-Bindungs-/anti-lymphoproliferativen Stelle der genannten Interferone entsprechen, mit dem Ziel, die Wirkungen der Immunsuppressiva zu verstärken, um ihre therapeutische Dosis zu reduzieren und somit ihre unerwünschten Nebenwirkungen während der Organ- oder Gewebetransplantation oder während der Behandlung verschiedener Krankheiten zu vermeiden, bei denen Cyclosporine, FK506 oder Rapamycin ausgenutzt werden können.

2. Zusammensetzung nach Anspruch 1, bestehend aus den Immunsuppressiva Cyclosporine, FK506 oder Rapamycin mit einem oder mehreren der Peptide, die mindestens 5 identische Aminosäurereste aus der folgenden Tabelle enthalten:
| | | | | | | | |
|---|---|---|---|---|---|---|---|
| L | T | E | K | K | Y | S | P |
| | R | R | R | R | H | R | R |
| | Q | D | N | D | | D | D |
| | M | L | M | N | | N | L |
| | | I | S | S | | S | |
| | E | A | E | | | A | |
| | G | | | | | | |
wobei,
jeder Aminosäurerest in der obersten Sequenz durch einen beliebigen Aminosäurerest darunter modifiziert werden kann.

3. Zusammensetzung nach Anspruch 1, bestehend aus den Immunsuppressiva Cyclosporine, FK506 oder Rapamycin und rekombinanten Proteinen, die eine oder mehrere Sequenzen tragen, die der Peptidvariante nach Anspruch 2 entsprechen.

4. Zusammensetzung nach Anspruch 2, bestehend aus den Immunsuppressiva Cyclosporine, FK506 und Rapamycin und den bioaktiven Peptiden, wobei die aktiven Peptide genetisch oder chemisch modifiziert oder genetisch oder chemisch oder physikalisch an einen niedermolekularen oder makromolekularen Stoff gebunden sind, um die Stabilität der Peptide unter physiologischen Bedingungen zu erhöhen oder die Bioverfügbarkeit der Peptide zu regulieren.

## Revendications

1. Composition médicamenteuse constituée des immunosuppresseurs cyclosporines, FK506 ou rapamycine, et des peptides bioactifs correspondant au site de liaison/antilymphoprolifératif à haute affinité des interférons α, β, ω, τ, ou de protéines recombinantes portant une ou plusieurs des séquences correspondant à la structure des peptides bioactifs correspondant au site de liaison/antilymphoprolifératif à haute affinité desdits interférons, dans le but d'amplifier l'activité des immunosuppresseurs, pour diminuer leur dose thérapeutique, et en conséquence pour éviter leurs effets secondaires indésirables pendant une transplantation d'organe et de tissu ou pendant le traitement de différentes maladies dans lesquelles des cyclosporines, du FK506 ou de la rapamycine peuvent être exploités.

2. Composition selon la revendication 1, constituée des immunosuppresseurs cyclosporines, FK506 ou rapamycine, ainsi que d'un ou plusieurs des peptides contenant au moins 5 résidus d'acides aminés identiques du tableau suivant :
| | | | | | | | |
|---|---|---|---|---|---|---|---|
| L | T | E | K | K | Y | S | P |
| | R | R | R | R | H | R | R |
| | Q | D | N | D | | D | D |
| | M | L | M | N | | N | L |
| | | I | S | S | | S | |
| | E | A | E | | | A | |
| | G | | | | | | |
dans lequel chaque résidu d'acide aminé de la séquence supérieure peut être modifié par l'un quelconque des résidus d'acides aminés situés en dessous.

3. Composition selon la revendication 1, constituée des immunosuppresseurs cyclosporines, FK506 ou rapamycine, et de protéines recombinantes portant une ou plusieurs des séquences correspondant à la variante de peptides de la revendication 2.

4. Composition selon la revendication 2, constituée des immunosuppresseurs cyclosporines, FK506 et rapamycine, et des peptides bioactifs, où les peptides bioactifs sont génétiquement ou chimiquement modifiés, ou génétiquement ou chimiquement ou physiquement liés à une substance à petite masse moléculaire ou macromoléculaire, dans le but d'augmenter la stabilité des peptides dans les conditions physiologiques, ou de réguler la biodisponibilité de ces peptides.
